(19) Europäisches Patentamt
European Patent Office
Office européen des brevets

(11) EP 2 119 494 A1

(12) # EUROPEAN PATENT APPLICATION
published in accordance with Art. 153(4) EPC

(43) Date of publication:
**18.11.2009 Bulletin 2009/47**

(21) Application number: **08704057.2**

(22) Date of filing: **29.01.2008**

(51) Int Cl.:
***B01D 69/08*** ^(2006.01) ***A61M 1/18*** ^(2006.01)
***B01D 63/02*** ^(2006.01) ***B01D 71/38*** ^(2006.01)
***B01D 71/44*** ^(2006.01) ***B01D 71/52*** ^(2006.01)
***B01D 71/68*** ^(2006.01)

(86) International application number:
**PCT/JP2008/051248**

(87) International publication number:
**WO 2008/093654 (07.08.2008 Gazette 2008/32)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.01.2007 JP 2007019019**

(71) Applicant: **Toray Industries, Inc.**
**Tokyo 103-8666 (JP)**

(72) Inventors:
- **OSABE, Masahiro**
  **Otsu-shi**
  **Shiga 520-8558 (JP)**
- **NAKAMATSU, Osamu**
  **Otsu-shi**
  **Shiga 520-8558 (JP)**
- **SUGAYA, Hiroyuki**
  **Otsu-shi**
  **Shiga 520-8558 (JP)**

(74) Representative: **Prüfer & Partner GbR**
**European Patent Attorneys**
**Sohnckestrasse 12**
**81479 München (DE)**

(54) **HOLLOW-FIBER MEMBRANE AND HOLLOW-FIBER-MEMBRANE MODULE HAVING THE SAME INCLUDED THEREIN**

(57) The present invention provides hollow fiber membranes and a hollow fiber membrane module having the same included therein, which even when highly filled, can reduce the size of the module and simultaneously maintain a crimped state enabling a good flow of a dialysate or buffer in the module by an easy process, **characterized in that** the hollow fiber membrane is provided with a crimp having a wavelength of 15 to 25 mm and a crimp angle determined by the wavelength and an amplitude of 0.09 to 0.32.

Fig. 3

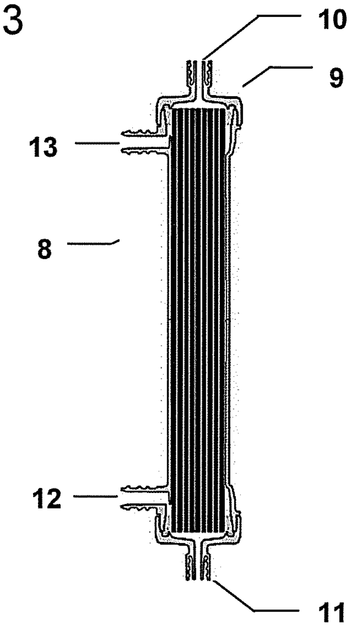


EP 2 119 494 A1

## Description

### Technical Field

The present invention relates to a hollow fiber membrane module. The present invention relates in particular to a hollow fiber membrane module having a hollow fiber dialyser or a built-in cross-flow filtration membrane used preferably in protein removal and fractionation.

### Background Art

A hollow fiber dialyser and a protein fractionation module fractionate waste matters, deleterious materials and proteins selectively from blood via a membrane by diffusion and filtration. The membrane is in the form of a flat membrane or a hollow fiber membrane, and in recent years, hollow fiber membranes are mainly used because of good processing efficiency and filtration performance.

Among the hollow fiber membranes, using the crossing flow type membrane, blood, a plasma or a serum flows in the hollow fiber membrane, and simultaneously a dialysate or buffer containing inorganic electrolytes etc. flows in the outside of the hollow fiber membrane,thereby removing object substances by diffusing them into the dialysate or buffer or by filtering them.

Among factors influencing this removal performance, there is the pore size of a membrane on which the performance of the hollow fiber membrane depends, and besides, the shape of a module and the flow of a dialysate are also important.

For example, when the number of hollow fibers in a module case is increased to highly fill the case with the fibers, a small case can be used, and lower costs of its material can be expected. However, when the case is highly filled, hollow fiber membranes in the module are adhered tightly to one another to reduce the mass transfer rate, thus reducing the diffusion performance of the module. This reduction in performance of the module exerts influences on reduction in the amount of intended substances removed from blood, plasma or serum.

As a method of improving the flow of a dialysate or buffer, a method wherein covering yarns are arranged as a spacer among hollow fiber membranes thereby preventing the follow fiber membranes from adhering to one another is known (Patent Documents 1 to 4).

When covering yarns are arranged to prevent adhesion among follow fiber membranes, the follow fiber membranes will not adhere to one another even after elapse of long periods. However, the step of arranging covering yarns is complicated and the packing density of hollow fiber membranes is reduced due to the presence of covering yarns. That is, the number of hollow fiber membranes filled in a module, and an effective membrane area (surface area in the hollow portion of a hollow fiber membrane (from which the area of an adhesive via which the hollow fiber membrane is bonded to a case in a module is removed)) $\times$ number of the hollow fiber membranes $\times$ length of the follow fiber membrane) is reduced, so that for securing the performance of the hollow fiber membranes as a module, the inner diameter of the module should inevitably be increased, and thus the cost of the hollow fiber membranes per module is increased.

On one hand, a method of giving a small wavy shape called crimp to hollow fiber membranes has been proposed as a method of preventing adhesion among hollow fiber membranes without reducing the packing density of the hollow fiber membranes.

With respect to the specific shape of crimp, modules having crimped hollow fiber membranes included therein wherein the diameter of a hollow fiber membrane and the wavelength and amplitude of crimp are defined (Patent Documents 5 and 6), the wavelength of crimp and the degree of crimping are defined (Patent Document 7), and crimpled hollow fiber membranes are partially used (Patent Document 8) have been proposed.

With respect to the method of preventing hollow fiber membranes from collapsing and flattening and simultaneously crimping the hollow fiber membranes, various methods have been proposed since the optimum method varies depending on the fiber diameter and material of the follow fiber membranes. For example, the following methods are disclosed: a method called a gear system wherein fibers are pressed into the space between two continuously rotating gears having mating teeth and simultaneously or subsequently heat-treated to fix crimping (Patent Document 9), a method of giving suitable stretching to fibers at ordinary temperature, thereby giving crimped fibers with fewer flat and heteromorphic fibers (Patent Document 10), a method of reeling fibers around a bobbin or the like and then heat-treating them at a temperature of 50°C or more, thereby fixing crimping (Patent Documents 11 and 12), and a method of delivering and simultaneously meandering lines of thread through a large number of thread guides at predetermined intervals and thermally fixing the lines of thread by heat-treatment (Patent Documents 13 and 14).

However, the methods described above were those providing crimp with a wavelength at intervals of 10 mm or less or 30 mm or more. In the case of the wavelength given at intervals of 10 mm or less, these methods are preferable from the viewpoint of improving performance of module, because the flow in a module can be improved even when the

crimp amplitude given to hollow fiber membranes is small. However, hollow fibers are subjected to physical loading of giving crimping with a short wavelength in a crimping step in membrane manufacturing, thus easily undergoing hollow fiber collapses during the process. Further, there is a problem that after winding, the bundle diameter is increased and the inner diameter of the module is increased so that costs become higher. When the bundle diameter is increased, there occurs not only the phenomenon in which costs become higher, but also the phenomenon in which the bundle when introduced into a module is hardly integrated in the module. In the case of the wavelength given at intervals of 30 mm or more, it is conceivable that the amplitude is increased to make the bundle diameter of the hollow fiber membrane large (andmake the membrane bulky) to a certain extent, thereby improving the flow of a dialysate or buffer to improve performance. In this case, however, unless the amplitude is significantly increased, bulkiness cannot be sufficiently changed, and thus the flow of a dialysate or buffer in the module cannot be made sufficiently uniform in some cases.

Patent Document 1: JP-B 59-18084
Patent Document 2: JP-A 60-244304
Patent Document 3: JP-A 02-140172
Patent Document 4: JP-A 04-2270
Patent Document 5: JP-A 57-194007
Patent Document 6: JP-B 5-12013
Patent Document 7: JP-A 62-266106
Patent Document 8: JP-A 64-22308
Patent Document 9: JP-A 09-021024
Patent Document 10: JP-A 2002-66274
Patent Document 11: JP-B 4-42022
Patent Document 12: JP-A 8-10322
Patent Document 13: JP-A 6-212520
Patent Document 14: JP-B 7-78293

## SUMMARY OF THE INVENTION

### Problems to be solved by the Invention

The present invention provides a hollow fiber membrane module which even when highly filled, can reduce the size of the module and simultaneously maintain a crimped state enabling a good flow of a dialysate or buffer in the module by an easy process to solve the drawback in the prior art.

### Means for solving the Problems

To solve the problem, the present invention is achieved by the following constitutions 1 to 12:

1. A hollow fiber membrane provided with a crimp having a wavelength of 15 to 25 mm and a crimp angle determined by the wavelength and an amplitude of 0.09 to 0.32.
2. The hollow fiber membrane according to item 1, wherein the flux of the hollow fiber membrane is $7.2 \times 10^{-10}$ $m^3/m^2/Pa/s$ to $25 \times 10^{-10}$ $m^3/m^2/Pa/s$.
3. The hollow fiber membrane according to item 1 or 2, wherein the hollow fiber membrane comprises a Polysulfone polymer.
4. The hollow fiber membrane according to any one of items 1 to 3, wherein the hollow fiber membrane comprises a water-soluble polymer.
5. The hollow fiber membrane according to any one of items 1 to 4, wherein the water-soluble polymer is any one of polyvinylpyrrolidone, polyethylene glycol and polyvinyl alcohol.
6. The hollow fiber membrane according to any one of items 1 to 5, whose outside surface rate of hole area is 6% or more.
7. The hollow fiber membrane according to any one of items 1 to 6, whose omission fall load is 0.05 N or less.
8. A hollow fiber membrane module comprising the hollow fiber membrane of any one of items 1 to 7 included therein.
9. The hollow fiber membrane module according to item 8, wherein the outer diameter of the hollow fiber membrane is 270 to 300 $\mu$m, and the fiber allocation in the cylindrical filter housing, of the hollow fiber membrane is 60 to 70% relative to the module case.
10. The hollow fiber membrane module according to item 8 or 9, wherein the amount of water in the hollow fiber membrane module is 200 to 350% relative to the weight of the hollow fiber membrane in a dried state.
11. The hollow fiber membrane module according to any one of items 8 to 10, wherein the volume of the module

case per $m^2$ of the hollow fiber membrane is $1.8 \times 10^4$ ($m^3/m^2$) or less.

12. A method for producing a hollow fiber membrane module, which comprises providing a hollow fiber membrane with a crimp having a wavelength of 15 to 25 mm and a crimp angle determined by the wavelength and an amplitude of 0.09 to 0.32, and incorporating the hollow fiber membrane into a module case.

Effect of the Invention

According to the present invention, there can be provided a hollow fiber membrane module which even when highly filled, can reduce the size of the module and simultaneously maintain a crimped state enabling a good flow of a dialysate or buffer in the module by an easy process.

BRIEF DESCRIPTION OF THE DRAWINGS

FIG. 1 shows measurement sites of wavelength and amplitude.
FIG. 2 shows a method of measuring a bundle diameter.
FIG. 3 shows a hollow fiber membrane module.
FIG. 4 is a sectional view of a crimp roll used in Example 1.
FIG. 5 shows a hollow fiber membrane crimped in Example 1.
FIG. 6 is a sectional view of a crimp roll used in Example 3.
FIG. 7 is a sectional view of a crimp roll used in Comparative Example 1.
FIG. 8 shows a hollow fiber membrane crimped in Comparative Example 1.
FIG. 9 is a sectional view of a crimp roll used in Comparative Example 2.
FIG. 10 shows a hollow fiber membrane crimped in Comparative Example 2.
FIG. 11 is a sectional view of a crimp roll used in Comparative Example 3.
FIG. 12 shows a hollow fiber membrane crimped in Comparative Example 3.

Description of the Symbols

1: crimped hollow fiber membrane
2: crimp wavelength
3: crimp amplitude
4: crimp angle θ
5: hexagonal hank
6: rolled bundle
7: weight at the time of measurement of bundle
8: module case
9: module header
10: first inlet
11: first outlet
12: second inlet
13: second outlet
14: ϕ6 cylindrical body
15: center distance of crimped roll
16: crimp roll central axis of cylindrical body
17: distance of radius 50 mm from the center of crimp roll central axis
18: R3 gear
19: ϕ8 cylindrical body
20: R1.4 gear

BEST MODE FOR CARRYING OUT THE INVENTION

The membrane material of the hollow fiber membrane of the present invention is not particularly limited, but is preferably a material used for medical purposes, and examples thereof include polyvinyl chloride, a cellulose polymer, polystyrene, polymethyl methacrylate, polycarbonate, polyurethane, polyacrylonitrile, and a Polysulfone polymer. Among

them, in particular, the Polysulfone polymer is easily molded, and when formed into a membrane, is excellent in material penetration performance, so a material containing the same is preferably used.

The Polysulfone polymer includes Polysulfone, polyether sulfones, and polyphenyl sulfones. Among them, the Polysulfon represented by the following chemical formulae (1) and (2) are preferably used. In the formulae, n is for example an integer of 50 to 80.

[Formula 1]

(1) 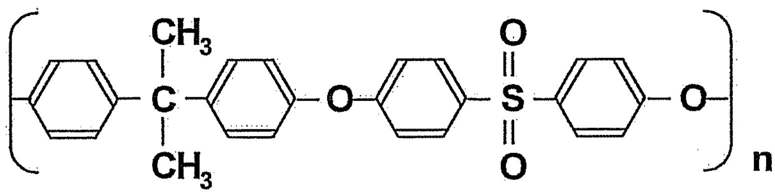

(2) 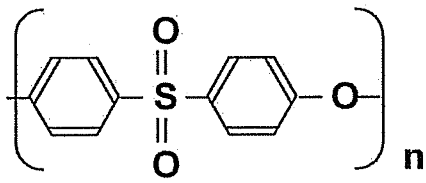

Specific examples of the polyarylsulfones include polysulfones such as Judel Polysulfons™ P-1700, P-3500 (manufactured by Solvay Advanced Polymers), Ladel™ A, R (manufactured by Solvay Advanced Polymers), Ultrazone S™ (manufactured by BASF), Ultrazone E™ (manufactured by BASF), and PEEK™ (Victrex). The polysulfones used in the present invention are preferably those polymers composed exclusively of repeating units represented by the formula (1) and/or (2), but may be copolymers with other monomers or derivatives having functional groups introduced into an aromatic ring, unless the effect of the present invention is impaired. It is preferable that the amount of such other copolymerization monomers in the composition is 10% by mole or less, and the proportion of derivative units in the repeating units is 10% by mole or less, but the invention is not limited thereto.

The polysulfone polymer is a hydrophobe polymer, but when the hydrophobe polymer is used and contacted with blood, serum or plasma, it is desirable to make the contact side a hydrophilicity. The hydrophilization method can be achieved by adding a water-soluble polymer to a spinning solution of a hollow fiber membrane or by applying a water-soluble polymer onto a contact surface with blood.

The water-soluble polymer referred to in the present invention refers to a polymer that can be dissolved in water. A water-soluble polymer having a weight-average molecular weight of 2000 or more is preferably used.

Specific examples of the water-soluble polymer include polyvinylpyrrolidone, polyethylene glycol, polyvinyl alcohol, etc.

The hollow fiber membrane module of the present invention is a module filled with the hollow fiber membranes described above, which has a function of removing waste matters or deleterious materials or fractionating intended proteins by penetration or diffusion by circulating and passing blood, serum or plasma through pores in the hollow fiber membranes. The process of the hollow fiber membrane module according to the present invention can be divided roughly into a process for producing hollow fiber membranes for cleaning blood and a process for integrating the hollow fiber membranes into a module.

As one example of the process for producing hollow fiber membranes, there is the following method. That is, it is preferable that polysulfones are dissolved in the range of 12 to 25% by weight in a spinning solution and then polyvinylpyrrolidone is dissolved in the range of 1 to 20% by weight in the spinning solution. When the amount of polysulfones is less than 12% by weight, the viscosity of the spinning solution is decreased, so that when continuous spinning is conducted, thread breakage and thread swaying spinning may occur to make spinning stability defective. When the amount of polysulfones is higher than 25% by weight, dimers contained in the polysulfone polymer may be

precipitated to make the spinning solution turbid. The spinning solution thus made turbid may cause clogging of a pipe and spinneret to make spinning difficult. Polyvinylpyrrolidone has an effect of both giving hydrophilicity and giving viscous increase to the spinning solution. Accordingly, when the amount of polyvinylpyrrolidone is less than 1% by weight, its effect of giving hydrophilicity to the spinning solution is not brought about, while the amount of polyvinylpyrrolidone is more than 20% by weight, the viscosity of the spinning solution is increased so that the melt fracture of the spinning solution upon discharge is caused. It is difficult for the melt fracture to control the hollow fiber membrane so as to attain a uniform shape. Polysulfones and polyvinylpyrrolidone, whendissolved, are mixed preferably with a good solvent for polysulfones (which is preferably N,N-dimethylacetamide, dimethylsulfoxide, dimethylformamide, N-methylpyrrolidone or dioxane) and further with 10% or less by weight of a poverty solvent for polysulfones (which is preferably water, polyethylene glycol or polypropylene glycol). When the content of a poor solvent in the spinning composition is 10% by weight or more, the spinning solution becomes turbid and may cause clogging in a pipe and spinneret to make spinning difficult. The spinning solution prepared by the method described above is discharged through a double annular spinneret. When discharged, an infusion liquid (a mixed solvent of good and poverty solvent for polysulfones) is passed through an inner pipe of the double spinneret, then delivered through a dry portion and brought into a coagulation bath. To prevent the influence of humidity in the dry portion, the outside surface of the resulting membrane running through the dry portion can be replenished with moisture, thereby accelerating cohesion of the polysulfones in the vicinity of the outside surface to enlarge the pore diameter, thus resulting in reduction in the resistance to penetration and diffusion during dialysis. The outside surface rate of hole area of the hollow fiber membrane after manufacturing can also be increased. Given too high relative humidity, however, coagulation of the spinning solution occurs predominantly in the outside surface of the membrane, thus reducing the pore diameter or forming a thin membrane, which results in a tendency to increase resistance to penetration and diffusion or in reduction of the outside surface rate of hole area in the membrane after manufacturing. Accordingly, the relative humidity is preferably 60 to 95%. The composition of the infusion solution is preferably based on the solvent used in the spinning solution, from the viewpoint of process adaptability. The infusion solution is preferably an aqueous solution wherein when dimethylacetamide for example is used, its concentration is 45% by weight or more, preferably 60% by weight or more and simultaneously 80% by weight or less, preferably 75% by weight or less. When the concentration is higher than 80% by weight or more, the coagulation rate of the hollow fiber membrane may be decreased to make membrane manufacturing infeasible.

Then, the hollow fiber membrane after passage through the coagulation bath is passed through a wash bath, thereby cleaning off a residual solvent and excessive hydrophilic polymers. Thereafter, the hollow fiber membrane is subjected to a drying step on line, followed by crimping the hollow fiber membrane. The method of successively crimping spun hollow fiber membranes includes (1) a method of passing a hollow fiber membrane between continuously rotating two gears having mating teeth, (2) a method of passing a hollow fiber membrane between a timing pulley and a timing belt, and (3) a method of passing a hollow fiber membrane between rugged belts. Inanymethods, the hollow fiber membranes in the crimping step are preferably in a tensioned state. This is because the hollow fiber membranes if subjected to tension are less liable to fiber disorder caused by spreading among the hollow fiber membranes and can be well crimped. However, too strong tension may cause fiber breakage etc., so attention is necessary. Such tension is preferably 3 gf or more, more preferably 4 gf or more, and even more preferably 5 gf or more and preferably 10 gf or less, more preferably 9 gf or less, and even more preferably 8 gf or less.

The wavelength of crimp in the present invention is 15 to 25 mm, preferably 18 to 22 mm. Preferably, the wavelength is periodically given. The periodical wavelength referred to the present invention shall mean that the crimp is given continuously such that the length of the crimp wavelength is within 10% within the average periodical wavelength. The crimp angle θ (rad) determined by the crimp wavelength and crimp amplitude is preferably 0.09 to 0.32, more preferably 0.12 to 0.28. From the viewpoint of improving performance of module, it is preferable that the crimp wavelength is less than 15 mm, or the crimp angle θ is less than 0.09, because a flow in the module can be improved even if the crimp amplitude given to the hollow fiber membranes is small. However, physical loading for giving the crimp with a short wavelength is given to the hollow fiber membranes in the crimping step in membrane manufacturing, so there is a problem that the hollow fiber membranes in this step are liable to hollow fiber collapses, the bundle diameter after winding is increased, the hollow fiber membranes when inserted into a module are hardly integrated in the module, and when the inner diameter of the module is increased, costs become higher. On the other hand, when the wavelength is more than 25 mm, the flow of a dialysate or buffer in the module may not be made sufficiently uniform. In this case, it is conceivable that the amplitude is increased to make the bundle diameter of the hollow fiber membrane large (and make the membrane bulky) to a certain extent, thereby improving the flow of a dialysate or buffer to improve performance, but unless the amplitude is significantly increased, bulkiness cannot be sufficiently changed, and so an improvement in the flow is difficult. When the crimp angle is larger than 0.32, there is an effect of improving the flow of a dialysate or buffer, but hollow fiber collapses may occur upon crimping. Hollow fiber collapses occur due to an increase in the contact area between crimping gears and the hollow fiber membrane and are caused by application of considerable loading to the hollow fiber membrane. Moreover, the bundle is made more bulky than necessary so that the bundle diameter is increased and the rate of successful insertion into a desired module case may be decreased. Even if successfully inserted, the

hollow fiber membranes may be cut due to fiber disorder and bent fibers in the module body. Crimping gears for giving crimp are hardly reduced in weight, thus increasing costs, and the width of the apparatus cannot be increased, thus making them unsuitable for mass production.

The means of giving crimp as described above is not particularly limited; for example, when the method (1) above is used, it is possible to use a means of appropriately regulating the pitch of mating teeth of a gear, the distance between two gears (mating distance), and the like.

The rate of successful insertion in the present invention is a possibility of success of insertion, into a module case, of hollow fiber membranes after winding in a hank. When the fiber bundle is thick, the hollow fiber membranes cannot be completely inserted into the case, or even when inserted into the case, may be broken. In this case, the rate of successful insertion is decreased.

As the method of giving crimp, a rod system makes use of crimping rolls each consisting of a $\phi 5$ to $\phi 10$ stainless steel cylindrical body or plated stainless steel rod and being arranged at equal spaces on a parallel in a longitudinal direction around a cylindrical body as a central axis. The material of the cylindrical body is not limited to the material described above. A cylindrical body of less than $\phi 5$ may "warp" due to the strength deficiency of the cylindrical body itself, so that when the crimp is given, the crimp with the above wavelength cannot be given to the hollow fiber membranes. A cylindrical body of more than $\phi 10$ may result in a higher cost thereof as a device and is not suitable for reduction in costs.

As the crimp wavelength or crimp angle $\theta$, the average value of 10 hollow fiber membranes arbitrarily selected from a fiber bundle removed from the module is used. When the fiber bundle is removed, the hollow fiber membranes shouldbe free fromtension as far as possible in order that the crimp shape is not changed, and for this purpose, the module case is cut with an ultrasonic disc cutter or the like.

The crimp wavelength and crimp angle $\theta$ are measured by the following method.

That is, the distance between the top of a peak and the top of a next peak shown in FIG. 1 is assumed to be a wavelength. For determining the crimp angle $\theta$, the crimp amplitude is first determined. The crimp amplitude is a numerical value obtained by connecting, with a straight line, two adjacent bottoms in an arbitrary position and then dividing, by 2, the length of a line vertically drawn from the top of a peak between the 2 bottoms to the straight line. In this manner, adj acent two peaks are measured for their amplitudes, and then the average of the 2 amplitudes thus measured is determined to provide one measured value. One hollow fiber membrane is subjected twice to this measurement to give two measured values, and the average of the two measured values is given as the measured value of the hollow fiber membrane (that is, 4 top-bottom distances are measured per hollow fiber membrane).

After the crimp amplitude and wavelength are determined in this manner, a sine curve drawn by the following equation is determined:

$$y = \text{amplitude} \times \sin((2 \times \text{circular constant}/\text{crimp wavelength} \times x)$$

wherein y is a variable indicative of amplitude, and x is a variable indicative of crimp wavelength.

A differential value of y:

$$dy/dx = \text{amplitude} \times 2 \times \text{crimp wavelength} \times \cos((2 \times \text{circular constant})/\text{crimp wavelength} \times x)$$

$$\text{Crimp angle } \theta \text{ (rad)} = \text{Tan}^{-1}(dy/dx)$$

In the present invention, as the outside surface rate of hole area of the hollow fiber membrane increases, the frictional properties of hollow fiber of the hollow fiber membrane decrease. This is because it can be considered that as the outside surface rate of hole area increases, the contact area decreases and the friction is decreases. Specifically, the outside surface rate of hole area is preferably 6% or more, more preferably 8% or more. On the other hand, when the outside surface rate of hole area is too large, membrane manufacturing performance and the rate of successful insertion into a module are reduced due to a deficiency of membrane strength and extensibility, and thus the outside surface rate of hole area is preferably 20% or less.

The outside surface rate of hole area is calculated by the following method.

First, the outside surface in an arbitrary position of the hollow fiber membrane is photographed at a magnification of 1000 times with a scanning electron microscope (for example, S-800 manufactured by Hitachi) and then subjected to image processing with Matrox Inspector 2.2 (Matrox Electronic Systems Ltd.). Specifically, pores in the outside surface are whitely reversed, the number of pores and the number of pixels in each pore are determined, and from the resolution of the image, the pore area is determined and the diameter of the pore is calculated. From these numerical values, their arithmetic mean value is determined as an average pore diameter. The outside surface rate is determined in percentage by dividing the total of pore areas by the image area.

As an indicator for evaluation of the frictional properties of hollow fiber, there is "omission fall load of hollow fiber membrane" determined by a method wherein one dried hollow fiber membrane is hanged on a stainless steel satin-finished round bar and then loaded at one end thereof with an increasing load, and the load with which the fiber slips down is determined as omission fall load. The measurement conditions of this method will be described in detail in the Examples. A smaller omission fall load is indicative of easier slipping, which indicates lower frictional properties of hollow fiber.

As a result of measurement of omission fall loads of hollow fiber membranes spun under various conditions, it was found that as the omission fall load of the hollow fiber membranes decreases, their bundle can be inserted more easily into the case. The omission fall load is preferably 0.05 N or less, more preferably 0.04N or less.

The outer diameter of the hollow fiber membrane is preferably 270 to 300 μm. When the inner diameter of the module case is the same, the distance between hollow fiber membranes is decreased as the outer diameter of the hollow fiber membranes is increased, and thus a dialysate or buffer hardly passes therethrough so that the removal performance of the membranes tends to decrease. There also arises a problem that the hollow fiber membrane bundle, when crimped, is increased and cannot be inserted into a module case. When the outer diameter of the hollow fiber membrane decreases, the hollow fiber membrane in the module case is subjected to lower fiber allocation in the cylindrical filter housing, so there is a problemthat a dialysate or buffer is liable to short pass. Accordingly, the outer diameter of the hollow fiber membrane is preferably 270 to 300 μm. The outer diameter of the hollow fiber membrane is the average diameters of 16 hollow fiber membranes picked out at random from the hollow fiber membrane bundle and measured with a laser displacement meter or a micro watcher. The fiber allocation in the cylindrical filter housing is determined from the outer diameter of the hollow fiber membranes by the following equation:

$$\text{Fiber allocation in cylindrical filter housing} = (((\text{outer diameter of hollow fiber membrane})^2 \times \text{number of hollow fiber membranes})/(\text{inner diameter of module case body})^2) \times 100\ (\%)$$

With respect to the outer diameter of the hollow fiber membrane and the fiber allocation in the cylindrical filter housing, it can thus be established that as the outer diameter of the hollow fiber membrane decreases, the fiber allocation in the cylindrical filter housing also decreases, while as the outer diameter of the hollow fiber membrane increases, the fiber allocation in the cylindrical filter housing also increases. Accordingly, the fiber allocation in the cylindrical filter housing is preferably 60 to 70%, more preferably 60 to 65%. By designing the module in this range, short pass hardly occurs so that diffusion performance is improved, and the resulting module is one wherein the hollow fiber membranes can be easily inserted into a module case.

In the present invention, a hollow fiber membrane having a flux of $7.2 \times 10^{-10}$ $m^3/m^2/Pa/s$ to $25 \times 10^{-10} m^3/m^2/Pa/s$ is preferably crimped. This is because it is considered that when the flux is less than $7.2 \times 10^{-10}$ $m^3/m^2/Pa/s$, diffusion performance is also decreased. It is estimated that the diffusion performance can be further improved by making the crimp wavelength lower than in the present invention, simultaneously increasing the crimp angle θ, and keeping the amplitude constant. In this case, however, the bundle diameter is increased at the time of high filling rate, so that the module case is thickened and the cost is increased. On the other hand, a flux of more than $25 \times 10^{-10}$ $m^3/m^2/Pa/s$ is not preferable either because of concern that spinning stability is deteriorated during manufacturing of hollow fiber membranes. Generally, the flux of the hollow fiber membrane increases as the cohesion of its major polymer becomes slow. According to the production method described above, however, hollow fiber membranes having the flux described above can be obtained. Slow cohesion can be said to be "weakness" in membrane manufacturing so that as

the flux is increased, stability in membrane manufacturing is lowered.

The flux is performance indicative of the penetration amount of water at 37°C through hollow fiber membranes and is expressed by the following equation:

$$\text{Flux } (m^3/m^2/Pa/s) = QW/(T \times A \times P)$$

wherein QW is a filtration amount ($m^3/s$), T is a processing time (s), P is a pressure (Pa), and A is an effective membrane area ($m^2$).

The method of incorporating the hollow fiber membranes in a module is not particularly limited. By way of example, the method is as follows. First, the follow fiber membranes in a dried state are cut in suitable length, and a necessary number of cut follow fiber membranes are bundled and then introduced into a cylindrical module. The dried state referred to herein is a state of the hollow fiber membranes having a moisture maintenance rate of 20% or less. The moisture maintenance rate is given by the calculating formula below. For determining the moisture maintenance rate, the hollow fiber membranes should be dried. The hollow fiber membranes are dried at 100°C, and when the rate of change in weight comes to be within 2% per hour drying, then drying is finished.

$$\text{Moisture maintenance rate} = (\text{weight of water in hollow fiber membrane}/\text{weight of hollow fiber membrane when drying is finished}) \times 100\ (\%)$$

The hollow fiber membrane bundle is inserted into a case and then temporarily capped at both ends, and a resin called a potting resin is charged into both ends of the hollow fiber membranes. The method of charging a potting resin into the module under rotation with a centrifuge is a method wherein the potting resin is transferred from the center of the module to ends by centrifugal force and then solidified. This method is preferable because the potting resin is uniformly charged. After the potting resin is solidified, both ends of the hollow fiber membranes are cut to open both the ends followed by attaching a module header thereto, to yield a hollow fiber membrane module.

The material of the module case and header includes, but is not limitedto, polycarbonate, polypropylene and polystyrene. The potting resin includes, but is not limited to, polyurethane resin and epoxy resin.

the module is then subjected to sterilization and a cross-linking treatment by irradiation with radiation. When the hollow fiber membranes in this step consist of dried fibers, a making-to-moist step of moistening the fibers is necessary. At this time, the rate with moisture is preferably 200 to 350%. The rate with moisture as used herein is calculated from a value obtained by dividing a rate (%) of the weight of the module after making-to-moist by the weight thereof before making-to-moist, which is multiplied by 100:

$$\text{Rate with moisture } (\%) = \text{weight of module after making-to-moist}/\text{weight of module before making-to-moist} \times 100$$

When the rate with moisture is less than 200, cross-linking treatment during exposure to radiation becomes insufficient, so that when the module is used, oozes may flow out. When the rate with moisture is higher than 350%, the weight of the module itself is increased to make handleability deficient.

Making-to-moist in the present invention is a step of filling the module with purified water and then with a nitrogen gas. As the making-to-moist method, there is a method wherein for example, the hollow fiber membrane module is filled with a liquid, and then this liquid is drawn out either by pressurization with a gas or by depressurization, thereby moistening hollow fiber membranes in the hollow fiber membrane module.

In the present invention, the volume of the module case per $m^2$ of the hollow fiber membrane is $1.8 \times 10^4$ ($m^3/m^2$) or less. This is expressed by the following equation:

$$\text{Module case volume } (m^3) = \text{sectional area of module case} \times \text{length of module case}$$

$$\text{Hollow fiber membrane area } (m^2) = \text{number of hollow fiber membranes} \times \text{inner diameter of hollow fiber membrane} \times \text{effective length of hollow fiber membrane} \times \text{crimp coefficient} \times \text{circular constant}$$

$$\text{Module case volume per } m^2 \text{ of hollow fiber membrane} = \text{module case volume/hollow fiber membrane area}$$

The effective length of the hollow fiber membrane is the length of the hollow fiber membrane included into the module case minus the adhesion portion (potting portion) between the module case and the hollow fiber membrane. The crimp coefficient is calculated according to the following equations:

$$\tan^{-1} (\text{crimp wavelength/crimp amplitude}) = \theta_1$$

$$\text{Crimp coefficient} = 1/\cos\theta_1$$

The volume of the module case per $m^2$ of the hollow fiber membrane is an indicator of compactification of the hollow fiber membrane module. Therefore, the volume of the module case is preferably $1.8 \times 10^4$ ($m^3/m^2$), more preferably $1.75 \times 10^4$ ($m^3/m^2$). When the volume of the module case is a numerical value of higher than $1.8 \times 10^4$ ($m^3/m^2$), the cost of the module case is increased, and when the hollow fiber membrane bundle is inserted into the case, there are lots of spaces so that the pressure loss is lowered while a dialysate is introduced and discharged, and by this phenomenon, the pressure throughout the hollow fiber membrane is not made uniform, and therefore, the hollow fiber membrane cannot be effectively used in application for removing unnecessary substances by diffusion and filtration. That is, as the above numerical value becomes lower, the hollow fiber membrane becomes advantageous to costs.
The performance of the module can be evaluated by the ability thereof to remove uremic substances such as urea and creatine, and as an indicator of this ability, urea clearance or the like can be used. This evaluation can be carried out for example based on dialyzer performance evaluation criteria published in September 1982 and edited by Japanese Society for Artificial Organs.
Hereinafter, the present invention will be described in more detail by reference to the Examples, but the present invention is not limited thereto.

Examples

Measurement methods used are described below.

1. Measurement of flux

A plastic module having an effective length (length of that portion of the hollow fiber membranes in a tube which is not covered with an adhesive) of 0.1 m was produced by introducing hollow fiber membranes through a cylindrical tube having openings at both ends, and then fixing both ends with an adhesive (referred to hereinafter as mini-module), and the hollow fiber membranes were filled therein with water, and a water pressure of $1.3 \times 10^4$ Pa was applied to the inlet side of the module, and the filtration amount of water flowing out, per unit time, to the outside of the membranes

was measured. This measurement was conducted at a water temperature kept at 37°C. The flux was calculated according to the following formula:

$$\text{Flux } (m^3/m^2/Pa/s) = QW/(T \times A \times P)$$

wherein QW is a filtration amount ($m^3$/s), T is a processing time (s), P is a pressure (Pa), and A is an effective membrane area ($m^2$).

2. Method of measuring the crimp amplitude, wavelength, and crimp angle θ

When the hollow fiber membrane bundle integrated in the case was to be pulled out from the case, the module case was cut with an ultrasonic disc cutter so as not to change the crimp shape, and then the hollow fiber membrane was pulled out. From the hollow fiber membranes thus pulled out, 10 membranes were picked out, and 18-cm arbitrary portions thereof were arranged on a black paper such that their amplitude was made maximum, and while attention was paid so as not to apply tension, both the ends (within 0.5 cm from both the ends) were fixed with Scotch tape™. A copy was made of this paper and then enlarged at a magnification of 4 times with a copier. In making the copy, attention was paid so as not to collapse or damage the hollow fiber membranes.

In the image magnified 4 times, the top of a peak and the peak of a next peak in an arbitrary position were connected with a line as shown in FIG. 1, and the length of this line was measured to determine wavelength.

The amplitude is a numerical value obtained by connecting, with a straight line, two adjacent bottoms in an arbitrary position and then dividing, by 2, the length of a line vertically drawn from the top of a peak between the 2 bottoms to the straight line. In this manner, adjacent two peaks are measured for their amplitudes, and then the average of the 2 amplitudes thus measured is determined to provide one measured value. One hollow fiber membrane is subjected twice to this measurement to give two measured values, and the average of the two measured values is given as the measured value of the hollow fiber membrane (that is, 4 top-bottom distances are measured per hollow fiber membrane).

The 10 hollow fiber membranes pulled out were subjected to this measurement, and their average value was determined. The average value was expressed in millimeters and rounded off to one decimal place, to determine wavelength and amplitude respectively.

After the crimp amplitude and wavelength were determined, the crimp amplitude and wavelength were substituted in the following equation to determine crimp angle θ.

$$y = \text{amplitude} \times \sin((2 \times \text{circular constant}/\text{crimp wavelength} \times x)$$

$$dy/dx = \text{amplitude} \times 2 \times \text{crimp wavelength} \times \cos((2 \times \text{circular constant})/\text{crimp wavelength} \times x)$$

$$\text{Crimp angle } \theta \text{ (rad)} = \text{Tan}^{-1} (dy/dx)$$

3. Method of measuring the diameter of the hollow fiber membranes

16 hollow fiber membranes picked out at random from the hollow fiber membrane bundle were measured with a laser displacement meter (LS5040T manufactured by KEYENCE). AT this time, because these fibers had been crimped, the fibers were stretched by applying tension and simultaneously measured. The outer diameters of the 16 hollow fiber membranes were measured to determine the average outer diameter of the hollow fiber membranes. In measurement of the thickness of the hollow fiber membranes and the inner diameter of the hollow fiber membranes were measured

with a lens of 1000 magnifications (VH-Z100 manufactured by KEYENCE) to determine the thickness of the hollow fiber membranes, and this membrane thickness was doubled and then subtracted from the outer diameter of the hollow fiber membranes to determine the inner diameter of the hollow fiber membranes.

4. Measurement of the fiber allocation in the cylindrical filter housing

After the average outer diameter of the hollow fiber membranes was determined, this average outer diameter was raised to the second power, then multiplied by the number of hollow fiber membranes integrated in the module, divided by the square of the inner diameter of the module case, and multiplied by 100, to determine the fiber allocation in the cylindrical filter housing, as follows:

$$\text{Fiber allocation in cylindrical filter housing} = (((\text{outer diameter of hollow fiber membrane})^2 \times \text{number of hollow fiber membranes})/(\text{inner diameter of module case body})^2) \times 100\ (\%)$$

5. Measurement of the outside surface rate of hole area in the hollow fiber membranes

The outside surface in an arbitrary position of the hollow fiber membranes was photographed at a magnification of 1000 times with a scanning electron microscope (S-800 manufactured by Hitachi). The taken image was 655 × 740 pixels in size. The image was then subjected to image processing with Matrox Inspector 2.2 (Matrox Electronic Systems Ltd.). The pore portions were whitely reversed and the other portions were blackly reversed, and the number of pixels in the white portions was determined. The total number of pixels in the pore portions (total pore area) was divided by the number of pixels in the whole image and expressed in percentage as the pore ratio as follows:

$$\text{Pore ratio } (\%) = (\text{total number of pixels in the pore portions})/(\text{number of pixels in the whole image}) \times 100$$

Because the resolution of the image was 0.140845 μm/pixel, the area S of the above electron microscope image was calculated to be 9615.2 $\mu m^2$.

6. Measurement of omission fall load of the hollow fiber membrane

One stainless steel satin-finished round bar of 40 mm in diameter and two polyacetal free-rolls of 12 mm in diameter were arranged at an embrace angle of 130°, and one dried hollow fiber membrane of 20 cm in length was hanged thereon and then loaded at one end thereof with an increasing load, and the load with which the fiber slips down was determined as omission fall load. The hollow fiber membrane subjected to measurement was dried at 60°C for 3 hours. During the measurement, a neutralization air was used in such a degree as not to sway the fiber, thereby eliminating the influence of static electricity. Different 10 hollow fiber membranes were subjected to the same measurement and their arithmetic average was determined as the measured value.

As the omission fall load increases, the fibers hardly slide thus indicating a large frictional coefficient, that is, indicating that a bundle of the fibers is bulky and is hardly inserted into the case.

6. Method of measuring the bundle diameter

The hollow fiber membranes after subjected to drying and crimping steps on line, while being subjected to a tension of 3 g per membrane, were rolled around a hexagonal hank and continued to be rolled until a desired area of the membranes was reached. After rolling, the resulting bundle was kept in a state rolled around the hank, and a finishing line of 20 cm in length having a weight of about 3 g was twisted around the bundle. Then, the position where the fishing line with the weight hanging vertically was initiated to be twisted around the bundle was marked and the site at which this position overlapped with the position of the finishing line twisted once around the bundle was marked, and the length

of the finishing line between the 2 marks was determined. This length was divided by the circular constant to determine the bundle diameter. This measurement method is shown in FIG. 2.

Bundle diameter (mm) = periphery (mm) of fiber bundle/circular constant

7. Method of measuring the number of collapsed fibers at the modulus edge side

Both ends of the hollow fiber membrane bundle were fixed with polyurethane resin, and that portions of the polyurethane resin ends that were protruded from both ends of the case were cut off to open the module edge sides, and the module edge sides were visually checked to confirm the collapsed state of the whole hollow fiber membranes integrated therein. At this time, the length in an arbitrary direction of an arbitrary section of the hollow fiber membranes, and the length of the section in a direction perpendicular to the above direction, were measured, and when the length of the longer axis was different by 20% or more from the length of the shorter axis, the hollow fiber membranes were judged to be collapsed fibers.

8. Measurement of rate with moisture

The rate with moisture was calculated from a value obtained by diving the weight of the module after making-to-moist by the weight thereof before making-to-moist, which is multiplied by 100:

Rate with moisture (%) =weight of module after making-to-moist/weight of module before making-to-moist × 100

9. Evaluation of performance of module

The performance of the module was evaluated with urea clearance as an indicator. This experiment was carried out under dialyzer performance evaluation criteria published in September 1982 and edited by Japanese Society for Artificial Organs. For this dialyzer performance evaluation, there are 2 methods, and this experiment was carried out with 0 mmHg TMP as a standard. The clearance was calculated using the following formula. When the membrane area is different, overall mass transfer coefficient is calculated from the clearance, from which the conversion of the area can be carried out.

$$\text{Clearance } C_L \text{ (ml/min)} = \{(CBi - CBo)/CBi\} \times Q_B$$

wherein CBi is the concentration of urea at the side of module inlet, CBo is the concentration of urea at the side of module outlet, and $Q_B$ is the amount of a liquid supplied to module (ml/min) 10. Calculation of module case volume per $m^2$ of hollow fiber membrane

The volume of the module case per $m^2$ of the hollow fiber membrane was calculated according to the following equation:

Module case volume ($m^3$) = sectional area of module case × length of module case

$$\text{Hollow fiber membrane area } (m^2) = \text{number of hollow fiber membranes} \times \text{Inner diameter of hollow fiber membrane} \times \text{effective length of hollow fiber membrane} \times \text{crimp coefficient} \times \text{circular constant}$$

$$\text{Module case volume } (m^3/m^2) \text{ per 1 } m^2 \text{ of hollow fiber membrane} = \text{module case volume/hollow fiber membrane area}$$

The effective length of the hollow fiber membrane is the length of the hollow fiber membrane included into the module case minus the adhesion portion (potting portion) between the module case and the hollow fiber membrane. The crimp coefficient is calculated according to the following equations:

$$\tan^{-1} (\text{crimp wavelength/crimp amplitude}) = \theta_1$$

$$\text{Crimp coefficient} = 1/\cos\theta_1$$

Example 1

16 parts by weight of polysulfones (Judel Polysulfone P-3500 manufactured by Solvay), 4 parts by weight of polyvinylpyrrolidone (K30 manufactured by ISP) and 2 parts by weight of polyvinylpyrrolidone (K90 manufactured by ISP) were added to and mixed with 1 part by weight of water and then dissolved under heating at 90°C for 10 hours. The resulting spinning solution was discharged thorough an orifice-type double cylindrical spinneret having an outer diameter of 0.35 mm and an inner diameter of 0.25 mm in the circular slit portion heated at 50°C and simultaneously a solution consisting of 63.2% by weight of dimethylacetamide and 36.8% by weight of water was discharged as a core fluid through the inside pipe, and the discharged liquid was passed through a dry space of 350 mm in length under 90% relative humidity and then passed through a coagulation bath at 40°C filled with a solution consisting of 15% by weight of dimethylacetamide and 85% by weight of water, a washing bath filled with water at 80°C and a drying treatment apparatus at 150°C in this order. The roller rate of the coagulation bath was 30 m/min. Then, the product was passed through crimping rolls of a crimping apparatus (section of the apparatus is shown in FIG. 4) provided with crimping rolls in a rod system having 20 SUS cylindrical bodies (diameter 100 mm and $\phi$6) arranged around a gear. The gear is provided therein with a cylindrical body as the central axis, and this cylindrical body and the SUS cylindrical bodies are arranged on a parallel in a longitudinal direction. The distance from the central axis of the crimping roll to the top of the SUS cylindrical body is 50 mm.

The distance between the centers of the thread-line left and right crimping rolls was 97 mm, and the temperature in an atmosphere of the crimping rolls was 80°C. After crimping, 7308 hollow fiber membranes were reeled with a tension of 3 g per hollow fiber membrane. At this time, the hollow fiber membranes have a wavy shape as shown in FIG. 5, and the membrane thickness was 46 $\mu$m, the inner diameter of the hollow fiber membrane was 189 $\mu$m, the outer diameter of the hollow fiber membrane was 281 $\mu$m, the crimp amplitude was 0.95 mm, the wavelength was 20.18 mm, the crimp angle $\theta$ was 0.288, and the bundle diameter was 34.7 mm. The outside surface rate of hole area in the hollow fiber membrane was 10.51%, and the omission fall load was 0.031 N. The bundle of these hollow fiber membranes was cut in suitable length to open an effective membrane area of 1.0 $m^2$ at both ends and introduced into a polypropylene module case of 30.6 mm in inner diameter having 2 liquid discharge openings near the ends of the body. At this time, the fiber allocation in the cylindrical filler housing was 62.6%. At this time, the length from one module edge side to the other module edge side was 237 mm. The module case volume calculated per $m^2$ of the hollow fiber membrane was 1.74 $\times$ $10^4$ ($m^3/m^2$).

Both ends of the hollow fiber membrane bundle were fixed with polyurethane resin, and the portions of the polyurethane resin ends that were protruded from the ends of the case were cut off to open the module edge sides. When the module edge sides were examined, there was no collapsed fiber. Thereafter, the module was moistened. That is, the second inlet 5 and the second outlet 6 were closed, and purified water was passed through the first outlet 4 to the first inlet 3. Thereafter, the first inlet 3 and the second inlet 5 were closed, and purified was introduced through

the first outlet 4, and purified water was passed until the second outlet 6. At this time, the flow rate of purified water was 500 ml/min. and passed for 1 minute.

Then, the liquid was removed, and a nitrogen gas was filled. That is, the first outlet 4 and the second outlet 6 were closed, and compressed air at 23°C was passed at a flow rate of 2NL/min. through the second inlet 5 for 15 seconds, to push out the filled water in the space in the second liquid passage. Thereafter, compressed air at 23°C was passed through the second inlet 5, and the pressure in the space in the second passage was kept in a pressurized state of 0.1 MPa, and in this state, the inlet was closed. The first outlet 4 was opened, and compression air at 23°C was passed from the first inlet 3 to the first outlet 4 at a flow rate of 2 NL/min. for 30 seconds, to push away purified water in the space in the first liquid passage. Then, nitrogen at 23°C was passed at a low rate of 2 NL/min. for 15 seconds from the first inlet 3 to the first outlet 4, and filled therein, and the first inlet 3 and the first outlet 4 were sealed with a cap. Thereafter, nitrogen at 23°C was passed at a flow rate of 2 NL/min. for 15 seconds from the second inlet 5 to the second outlet 6, and filled therein, and the second inlet 5 and the second outlet 6 were sealed with a cap. Thereafter, γ-ray sterilization with an irradiation dose of 25 kGy was carried out. The rate with moisture was 280%.

As the hollow fiber membrane performance, the flux was $1.55 \times 10^{-9}$ $m^3/m^2/pa/s$, and urea clearance when measured as the performance of module was 188 ml/min.

Example 2

9600 hollow fiber membranes manufactured until the drying and crimping steps in the same method as in Example 1 were reeled. The bundle of these hollow fiber membranes was cut in suitable length to open an effective membrane area of 1.6 $m^2$ at both ends and introduced into a polypropylene module case of 35.1 mm in inner diameter having 2 liquid discharge openings near the ends of the body. At this time, the fiber allocation in the cylindrical filler housing was 62.0%. At this time, the length from one module edge side to the other module edge side was 285 mm. The module case volume calculated per $m^2$ of the hollow fiber membrane was $1.72 \times 10^4$ ($m^3/m^2$).

When the module edge sides were examined in the same manner as in Example 1, there was no collapsed fiber. Thereafter, the module was moistened in the same manner as in Example 1 and subjected to γ-ray sterilization with an irradiation dose of 25 kGy. The rate with moisture was 270%.

As the hollow fiber membrane performance, the flux was $1.55 \times 10^{-9}$ $m^3/m^2/pa/s$, and urea clearance when measured as the performance of module was 196 ml/min.

Example 3

The same composition as in Example 1 was dissolved at 90°C over 6 hours. The resulting spinning solution was discharged through an orifice-type double cylindrical spinneret having an outer diameter of 0.35 mm and an inner diameter of 0.25 mm in the circular slit portion heated at 50°C and simultaneously a solution consisting of 65.3% by weight of dimethylacetamide and 34.8% by weight of water was discharged as a core fluid through the inside pipe, and the discharged liquid was passed through a dry space of 350 mm in length under 80% relative humidity and then passed through a coagulation bath at 40°C filled with a solution consisting of 15% by weight of dimethylacetamide and 85% by weight of water, a washing bath filled with water at 90°C and a drying treatment apparatus at 125°C in this order. The roller rate of the coagulation bath was 28.5 m/min. Then, the product was passed through crimping rolls of a crimping apparatus (section of the apparatus is shown in FIG. 6) provided with crimping rolls having a diameter of 100 mm and 20 R3 gear teeth arranged at regular intervals on a circumference with a radius of 50 mm. The SUS cylindrical bodies are arranged on a parallel in a longitudinal direction. The distance from the central axis of the crimping roll to the top of the SUS cylindrical body was 50 mm.

The distance between the centers of the thread-line left and right crimping rolls was 92.5 mm, and the temperature in an atmosphere of the crimping rolls was 40°C. After crimping, 9600 hollow fiber membranes were reeled with a tension of 3.5 g per hollow fiber membrane. At this time, the membrane thickness was 40 μm, the inner diameter of the hollow fiber membrane was 200 μm, the outer diameter of the hollow fiber membrane was 280μ m, the crimp amplitude was 0.60 mm, the wavelength was 22.4 mm, the crimp angle θ was 0.167, and the bundle diameter was 37.4 mm. The outside surface rate of hole area in the hollow fiber membrane was 6.3%, and the omission fall load was 0.032 N. The bundle of these hollow fiber membranes was cut in suitable length to open an effective membrane area of 1.0 $m^2$ at both ends and introduced into a polypropylene module case of 35.1 mm in inner diameter having 2 liquid discharge openings near the ends of the body. At this time, the fiber allocation in the cylindrical filler housing was 62.0%. At this time, the length from one module edge side to the other module edge side was 285 mm. The module case volume calculated per $m^2$ of the hollow fiber membrane was $1.72 \times 10^4$ ($m^3/m^2$).

The module wax prepared in the same manner as in Example 1, and the rate with moisture was 260%.

As the hollow fiber membrane performance, the flux was $2.08 \times 10^{-9}$ $m^3/m^2/pa/s$, and urea clearance when measured as the performance of module was 195 ml/min.

Comparative Example 1

Membranes were manufactured under the same conditions until the drying step as in Example 1, and then the product was passed through crimping rolls of a crimping apparatus (section of the apparatus is shown in FIG. 7) provided with crimping rolls having 12 SUS cylindrical bodies having a diameter of 100 mm and ϕ8 arranged at regular intervals on a circumference with a radius of 50 mm. The distance between the centers of the thread-line left and right crimping rolls was 91.6 mm, and the temperature in an atmosphere of the crimping rolls was 80°C. After crimping, 7328 hollow fiber membranes were reeled with a tension of 3 g per hollow fiber membrane. At this time, the hollow fiber membranes have a wavy shape as shown in FIG. 8, and the membrane thickness was 39 μm, the inner diameter of the hollow fiber membrane was 200 μm, the outer diameter of the hollow fiber membrane was 279 μm, the crimp amplitude was 1.48 mm, the wavelength was 32.46 mm, the crimp angle θ was 0.265, and the bundle diameter was 40.8 mm. The outside surface rate of hole area in the hollow fiber membrane was 11.3%, and the omission fall load was 0.034 N. The bundle of these hollow fiber membranes was introduced into the same module case as in Example 1. At this time, the fiber allocation in the cylindrical filler housing was 61.9%. At this time, the length from one module edge side to the other module edge side was 237 mm. The module case volume calculated per $m^2$ of the hollow fiber membrane was $1.74 \times 10^4$ ($m^3/m^2$).

When the module edge sides were examined in the same manner as in Example 1, there was no collapsed fiber. Thereafter, the module was moistened in the same manner as in Example 1 and subjected to γ-ray sterilization with an irradiation dose of 25 kGy. The rate with moisture was 275%.

As the hollow fiber membrane performance, the flux was $1.34 \times 10^{-9}$ $m^3/m^2/pa/s$, and urea clearance when measured as the performance of module was 178 ml/min.

Comparative Example 2

Membranes were manufactured under the same conditions until the drying step as in Example 1, and then the product was passed through crimping rolls of a crimping apparatus (FIG. 9) provided with crimping rolls having 16 SUS cylindrical bodies having a diameter of 100 mm and ϕ8 arranged at regular intervals on a circumference with a radius of 50 mm. The distance between the centers of the thread-line left and right crimping rolls was 97 mm, and the temperature in an atmosphere of the crimping rolls was 80°C. After crimping, 7344 hollow fiber membranes were reeled with a tension of 3 g per hollow fiber membrane. At this time, the hollow fiber membranes have a wavy shape as shown in FIG. 10, and the membrane thickness was 47 μm, the inner diameter of the hollow fiber membrane was 187 μm, the outer diameter of the hollow fiber membrane was 281 μm, the crimp amplitude was 1.08 mm, the wavelength was 25.66 mm, the crimp angle θ was 0.259, and the bundle diameter was 38.4 mm. The outside surface rate of hole area in the hollow fiber membrane was 10.89%, and the omission fall load was 0.032 N. The bundle of these hollow fiber membranes was introduced into the same module case as in Example 1. At this time, the fiber allocation in the cylindrical filler housing was 62.7%. At this time, the length from one module edge side to the other module edge side was 237 mm. The module case volume calculated per $m^2$ of the hollow fiber membrane was $1.74 \times 10^4$ ($m^3/m^2$).

When the module edge sides were examined in the same manner as in Example 1, there was no collapsed fiber. Thereafter, the module was moistened in the same manner as in Example and subj ected to γ-ray sterilization with an irradiation dose of 25 kGy. The rate with moisture was 285%.

As the hollow fiber membrane performance, the flux was $1.24 \times 10^{-9}$ $m^3/m^2/pa/s$, and urea clearance when measured as the performance of module was 184 ml/min.

Comparative Example 3

Membranes were manufactured under the same conditions until the drying step as in Example 1, and then the product was passed through crimping rolls of a crimping apparatus (FIG. 11) provided with crimping rolls having a diameter of 100 mm and 50 R31.4 gear teeth arranged at regular intervals on a circumference with a radius of 50 mm. The distance between the centers of the thread-line left and right crimping rolls was 10 mm, and the temperature in an atmosphere of the crimping rolls was 80°C. After crimping, 7216 hollow fiber membranes were reeled with a tension of 3 g per hollow fiber membrane. At this time, the hollow fiber membranes have a wavy shape as shown in FIG. 12, and the membrane thickness was 47 μm, the inner diameter of the hollow fiber membrane was 189 μm, the outer diameter of the hollow fiber membrane was 279 μm, the crimp amplitude was 0.44 mm, the wavelength was 6.78 mm, the crimp angle θ was 0.387, and the bundle diameter was 42.1 mm. The outside surface rate of hole area in the hollow fiber membrane was 9.15%, and the omission fall load was 0.038 N. The bundle of these hollow fiber membranes was introduced into the same module case as in Example 1. At this time, the fiber allocation in the cylindrical filler housing was 61.9%. At this time, the length from one module edge side to the other module edge side was 237 mm. The module case volume calculated per $m^2$ of the hollow fiber membrane was $1.74 \times 10^4$ ($m^3/m^2$).

When the module edge sides were examined in the same manner as in Example 1, there were 90 collapsed fibers. Thereafter, the module was moistened in the same manner as in Example 1 and subjected to γ-ray sterilization with an irradiation dose of 25 kGy. The rate with moisture was 280%.
As the hollow fiber membrane performance, the flux was $1.26 \times 10^{-9}$ $m^3/m^2/pa/s$, and urea clearance when measured as the performance of module was 188 ml/min.

**Table 1**

| | Fiber diameter ($\mu m$) | | | Crimp | | | Outside surface rate of hole area | Omission fall load | Flux |
|---|---|---|---|---|---|---|---|---|---|
| | Film thickness | Inner diameter | Outer diameter | Amplitude (mm) | Wavelength (mm) | $\theta$ (rad) | (%) | (N) | ($m^3/m^2/Pa/s$) |
| Example 1 | 46 | 189 | 281 | 0.95 | 20.18 | 0.288 | 10.51 | 0.031 | $1.55\times10^{-9}$ |
| Example 2 | 46 | 189 | 281 | 0.95 | 20.18 | 0.288 | 10.51 | 0.031 | $1.55\times10^{-9}$ |
| Example 3 | 40 | 200 | 280 | 0.60 | 22.40 | 0.167 | 6.3 | 0.032 | $2.08\times10^{-9}$ |
| Comparative Example 1 | 39 | 200 | 279 | 1.48 | 32.46 | 0.265 | 11.3 | 0.034 | $1.34\times10^{-9}$ |
| Comparative Example 2 | 47 | 187 | 281 | 1.08 | 25.66 | 0.259 | 10.89 | 0.032 | $1.24\times10^{-9}$ |
| Comparative Example 3 | 47 | 189 | 279 | 0.44 | 6.78 | 0.387 | 9.15 | 0.038 | $1.26\times10^{-9}$ |

**Table 2**

| | Effective area | Fiber allocation in cylindrical filter housing | MD volume per $m^2$ of hollow fiber membrane | Rate with moisture |
|---|---|---|---|---|
| | ($m^2$) | (%) | ($m^3/m^2$) | (%) |
| Example 1 | 1.0 | 62.6 | $1.74 \times 10^4$ | 280 |
| Example 2 | 1.6 | 62.0 | $1.72 \times 10^4$ | 270 |
| Example 3 | 1.6 | 62.0 | $1.72 \times 10^4$ | 260 |
| Comparative Example 1 | 1.0 | 61.9 | $1.74 \times 10^4$ | 275 |
| Comparative Example 2 | 1.0 | 62.7 | $1.74 \times 10^4$ | 285 |
| Comparative Example 3 | 1.0 | 61.9 | $1.74 \times 10^4$ | 280 |

## Claims

**1.** A hollow fiber membrane provided with a crimp having a wavelength of 15 to 25 mm and a crimp angle determined by the wavelength and an amplitude of 0.09 to 0.32.

**2.** The hollow fiber membrane according to claim 1, wherein the flux of the hollow fiber membrane is $7.2 \times 10^{-10}$ $m^3/m^2/Pa/s$ to $25 \times 10^{-10}$ $m^3/m^2/Pa/s$.

**3.** The hollow fiber membrane according to claim 1 or 2, wherein the hollow fiber membrane comprises a polysulfone polymer.

**4.** The hollow fibermembrane according to any one of claims 1 to 3, wherein the hollow fiber membrane comprises a water-soluble polymer.

**5.** The hollow fiber membrane according to any one of claims 1 to 4, wherein the water-soluble polymer is any one of polyvinylpyrrolidone, polyethylene glycol and polyvinyl alcohol.

**6.** The hollow fiber membrane according to any one of claims 1 to 5, whose outside surface rate of hole area is 6% or more.

**7.** The hollow fiber membrane according to any one of claims 1 to 6, whose omission fall load is 0.05 N or less.

**8.** A hollow fiber membrane module comprising the hollow fiber membrane of any one of claims 1 to 7 included therein.

**9.** The hollow fiber membrane module according to claim 8, wherein the outer diameter of the hollow fiber membrane is 270 to 300 μm, and the fiber allocation in the cylindrical filter housing, of the hollow fiber membrane is 60 to 70% relative to the module case.

**10.** The hollow fiber membrane module according to claim 8 or 9, wherein the amount of water in the hollow fiber membrane module is 200 to 350% relative to the weight of the hollow fiber membrane in a dried state.

**11.** The hollow fiber membrane module according to any one of claims 8 to 10, wherein the volume of the module case per $m^2$ of the hollow fiber membrane is $1.8 \times 10^4$ ($m^3/m^2$) or less.

**12.** A method for producing a hollow fiber membrane module, which comprises providing a hollow fiber membrane with a crimp having a wavelength of 15 to 25 mm and a crimp angle determined by the wavelength and an amplitude of 0.09 to 0.32, and incorporating the hollow fiber membrane into a module case.

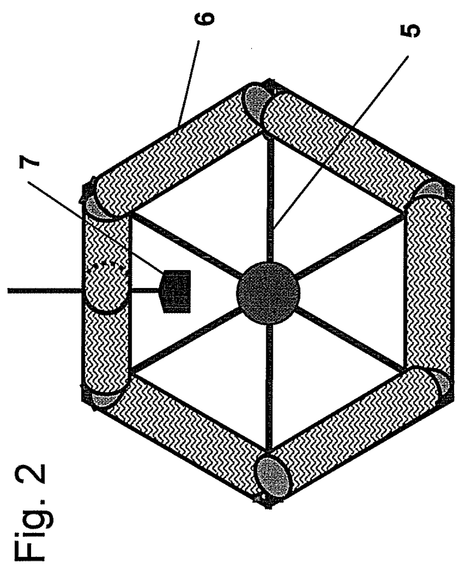


Fig. 2

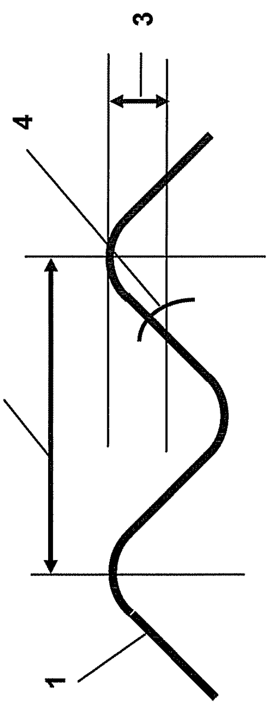


Fig.1

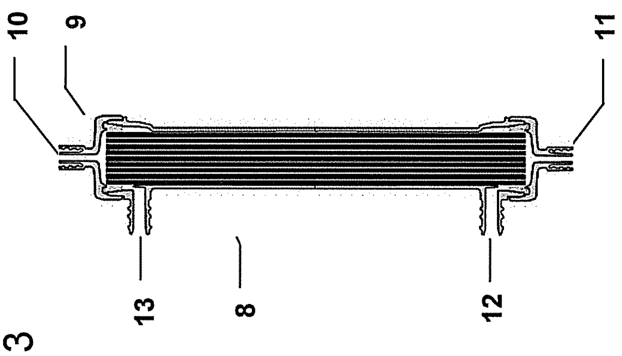


Fig. 3

Fig. 4

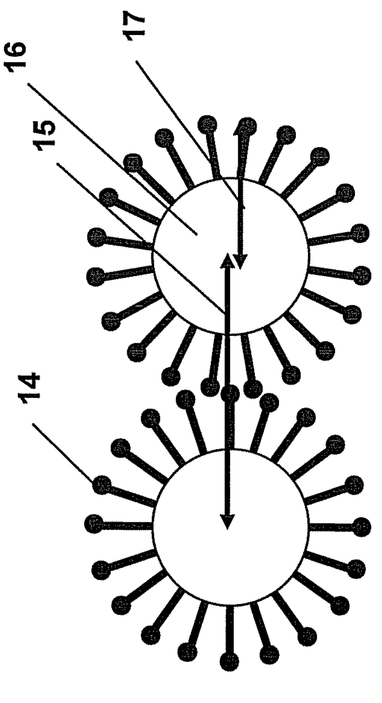


Fig. 5

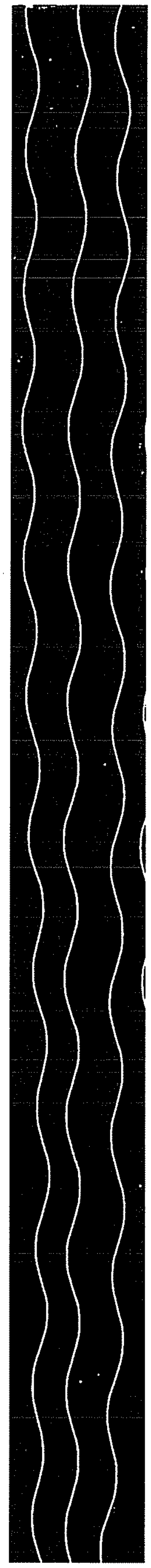

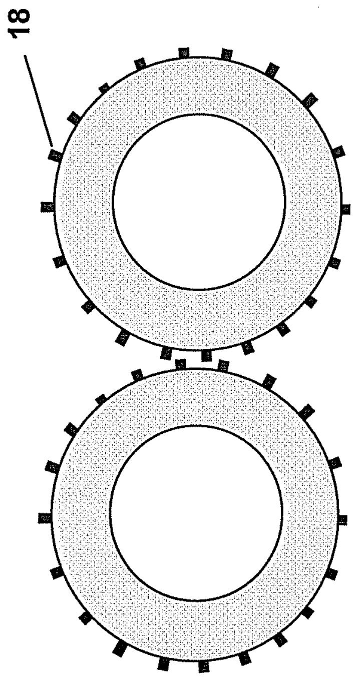


Fig. 6

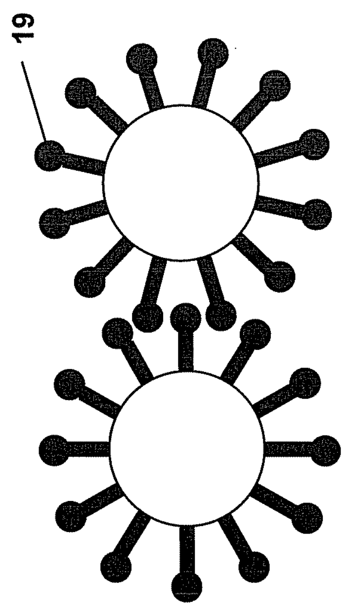


Fig. 7

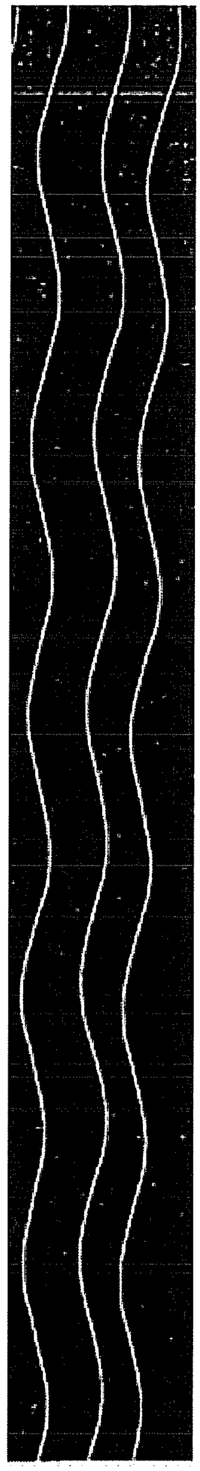

Fig. 8

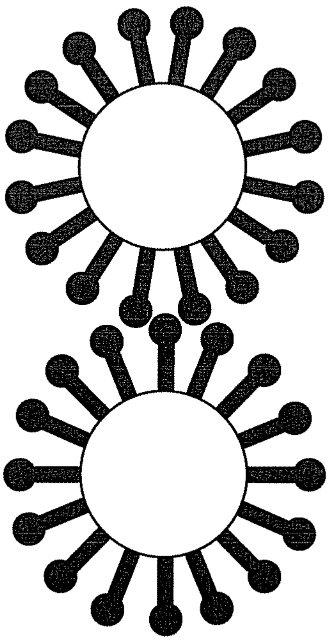

Fig. 9

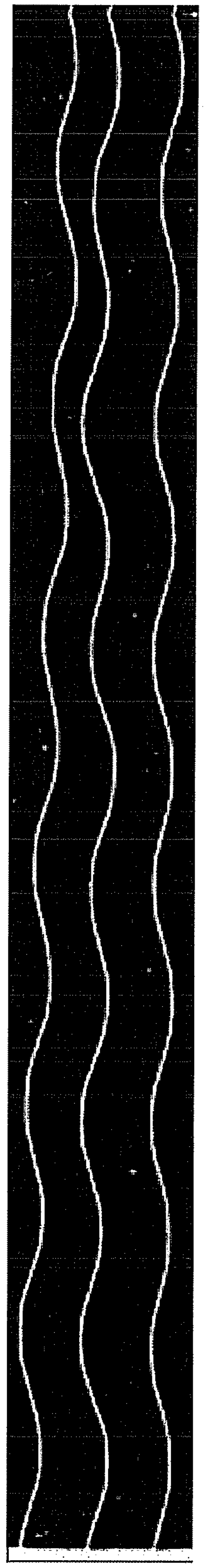

Fig. 10

Fig. 11

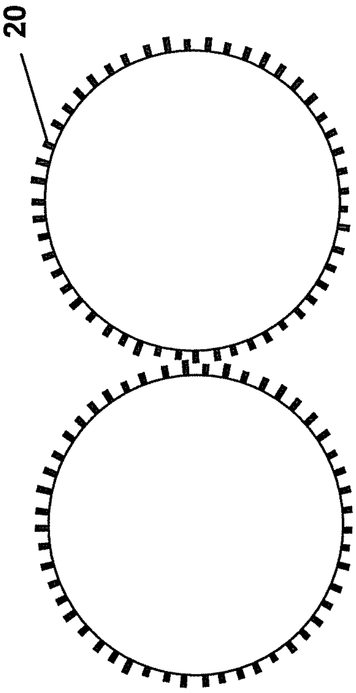


Fig. 12

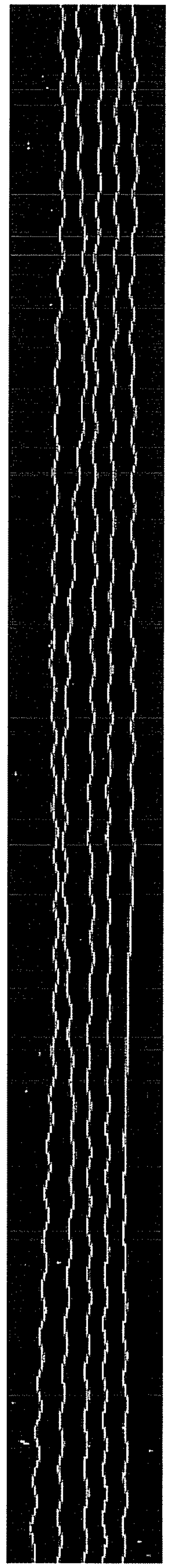

## INTERNATIONAL SEARCH REPORT

International application No.
PCT/JP2008/051248

A. CLASSIFICATION OF SUBJECT MATTER
*B01D69/08*(2006.01)i, *A61M1/18*(2006.01)i, *B01D63/02*(2006.01)i, *B01D71/38*(2006.01)i, *B01D71/44*(2006.01)i, *B01D71/52*(2006.01)i, *B01D71/68*(2006.01)i

According to International Patent Classification (IPC) or to both national classification and IPC

B. FIELDS SEARCHED

Minimum documentation searched (classification system followed by classification symbols)
B01D61/00-71/82, A61M1/00-1/38, C02F1/44

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
WPI

C. DOCUMENTS CONSIDERED TO BE RELEVANT

| Category* | Citation of document, with indication, where appropriate, of the relevant passages | Relevant to claim No. |
|---|---|---|
| X<br>Y | JP 10-57776 A (Toyobo Co., Ltd.),<br>03 March, 1998 (03.03.98),<br>Particularly, Claims; Par. Nos. [0007], [0010]; example 6<br>(Family: none) | 1,3,7,8,12<br>2,4-6,9-11 |
| X<br>A | JP 9-266947 A (Teijin Ltd.),<br>14 October, 1997 (14.10.97),<br>Comparative example 2<br>(Family: none) | 1,8,12<br>2-7,9-11 |
| X<br><br>Y | JP 2006-288415 A (Toyobo Co., Ltd.),<br>26 October, 2006 (26.10.06),<br>Particularly, Claims; examples;<br>Par. Nos. [0091] to [0095], [0119]<br>(Family: none) | 1-5,7-9,11,<br>12<br>6,10 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 February, 2008 (22.02.08) | 11 March, 2008 (11.03.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2008/051248

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 2005-334566 A (Toyobo Co., Ltd.),<br>08 December, 2005 (08.12.05),<br>Particularly, Claims; Par. Nos. [0031], [0042], [0050]<br>(Family: none) | 2-6,8,9<br>1,7,10,11,12 |
| Y<br>A | JP 2006-198611 A (Toray Industries, Inc.),<br>03 August, 2006 (03.08.06),<br>Particularly, Claims; Par. No. [0013]<br>(Family: none) | 3-5,8,10<br>1,2,6,7,9, 11,12 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 59018084 B
- JP 60244304 A
- JP 2140172 A
- JP 4002270 A
- JP 57194007 A
- JP 5012013 B
- JP 62266106 A
- JP 6422308 A
- JP 9021024 A
- JP 2002066274 A
- JP 4042022 B
- JP 8010322 A
- JP 6212520 A
- JP 7078293 B